# EUROPEAN PATENT APPLICATION

(11) **EP 3 412 206 A1**
(43) Date of publication of application: **12.12.2018**
(21) Application number: 17175293.4
(22) Date of filing: 09.06.2017
(51) Int. Cl.: A61B 5/20, G01G 23/00, G01G 17/04, G01G 21/22, G01G 3/14, G01G 19/56, A61B 10/00

(54) **PORTABLE URINATION WEIGHT MEASUREMENT DEVICE**

(71) Applicant: Centre Hospitalier Universitaire de Liège, 4000 Liège (BE); WSL, 4031 Liège (BE)
(72) Inventor: JOURET, François, 4053 Embourg (BE); BIHR, Damien, 1190 Forest (BE); NICLAES, Christophe, 5300 Vezin (BE)
(74) Representative: DenK iP

(57) **Abstract**

A portable urination weight measurement device comprising:
- a holding unit for releasably holding a container, and
- a handle comprising a weight measurement unit coupled to the holding unit so as to allow the measure of a weight exerted on the holding unit; and a toilet flushable container having:
∘ a rim having a fold at a front side of the opening and a fold at a back side of the opening, each fold forming at rest an angle of less than 20°, and
∘ a collar secured to the rim and comprising at least one fold at the front side of the opening forming at rest an angle of less than 20°, the collar being of a rigidity such that increasing the angle of the collar fold to 180° increases the angle of the rim fold at the back of the opening to from 50 to 90°.

## Description

### Technical field of the invention

The present invention relates to the field of medical apparatuses, and in particular relates to a portable urination weight measurement device and to a container for use therewith.

### Background of the invention

The diagnosis and follow-up of various human diseases require the characterization of urine volume and composition. Classically, urine is analyzed either on spot samples or after an entire 24-hour collection. The cohort of patients includes for example patients with history of kidney stones, patients with autosomal dominant polycystic kidney disease, patients under chronic dialysis, patients with chronic kidney disease, patients with chronic heart failure, patients with chronic liver failure, women following perineal physiotherapy after delivery, men following vesical reeducation after surgery, amongst others. In addition, a significant proportion of individuals complain about pollakiuria (increased frequency of mictions), polyuria (excessive volume of urine) or nycturia (urine evacuation during the night). The veracity and severity of these symptoms are (very) difficult to assess in clinical routine. Finally, beside medical applications, one may envision personal use of such a device since more and more individuals collect more and more data about their own body (weight, height, heart rate, number of steps per day, sleep movements, ingested calories, etc...). Accurate and easy-to-obtain information about daily urine production would add significant data to this health check-up list.

The evaluation of the daily production of urine (i.e. the volume of urine produced in 24 hours, also called "*diuresis*") in both terms of volume and frequency does not necessarily require urine collection and storage. Nowadays, the patients are asked to collect their urine for 24 hours, at home or at the hospital, in appropriate (clean) containers and bring them to the lab for volume assessment. This represents a major source of errors and evident hygienic inconveniences for both male and female, young and elderly, patients. Furthermore, the information about miction frequency is most often lacking since most patients do not record when they urinate.

Known alternatives to measure urine volume are:
- to urinate in a graduated container and record the value; and
- to urinate in a container, weight the urine-filled container (after deducting the tare of the container) and record the value.

In patent application WO201321207, a urine flow measuring apparatus is described, that comprises a controller to automatically measure and record a weight of urine. However, this device shows multiple limitations, including (i) the need for non-hygienic manipulations when emptying the container into the toilets and when cleaning the container, (ii) the difficulty to carry it from place to place (since the patient is supposed to measure 24-hour urine production), (iii) the poor ergonomics in sitting position, (iv) the malposition of the handle (with regards to urine orientation).

CN105496429 discloses a portable urination recorder comprising a hand shank and a urine storage cavity, wherein one end of the hand shank is fixedly connected with the urine storage cavity; the urine storage cavity is in a cavity structure with openings in two ends; the diameter of the opening in one end is greater than that in the other end; a urine volume detection device and an integrated circuit chip are arranged in the urine storage cavity; the urine volume detection device is electrically connected with the integrated circuit chip; a data transmission device is arranged in the hand shank; and the data transmission device is electrically connected with the integrated circuit chip. The portable urination recorder has the beneficial effects that automatic monitoring of urination can be carried out; and the urination measurement accuracy is allegedly improved. This system has however the major drawback that the urine storage cavity must be washed after each use, which is an activity that often cannot be performed in the privacy of a WC booth. Furthermore, the urine storage cavity is rigid and cumbersome so that it takes an exaggerate amount of place in a handbag. Additionally, any reduction in bulkiness of the urine storage cavity translates in an increased risk of urine landing outside of the urine storage cavity, and hence in an inaccurate urination measurement.

There is therefore still a need in the art for apparatus improving on one or more of the above drawbacks.

### Summary of the invention

It is an object of the present invention to provide good apparatus or methods for measuring the weight of an urination.

It is an advantage of embodiments of the present invention that they may be portable. The portable urination weight measurement device can be made small enough to be carried in a handbag or a pocket. The containers may be folded flat when not in use. The portable urination weight measurement device can also be folded so as to take minimum space when not in use. Furthermore, the portable urination weight measurement device may weigh less than 500g.

It is an advantage of embodiments of the present invention that they may be ergonomic. The portable urination weight measurement device can be made with dimensions making it very suitable for being placed between the legs of the user during use. Also, the shape of the container has a shape which also accommodates well the presence of the legs of the user. The portable urination weight measurement device may also be used in standing position.

It is an advantage of embodiments of the present invention that they are accurate. The shape, dimensions and position of the container during use may typically permit every drop of urine to be collected for both male and female. Also, due to the measurement of a weight instead of the direct measurement of a volume, which might depend on temperature and on changes in the shape of the container, systematic errors are more easily avoided. Also, in embodiments, the device may take into account tilt angle or movements of the device during use to correct the measured weight or to record a measure as faulty.

It is an advantage of embodiments of the present invention that they are precise. A precision of 1g or even 0.1g can easily be achieved.

It is an advantage of embodiments of the present invention that they are hygienic. Indeed, the user never has to touch urine or the urine-filled container since the container is mechanically released in the toilet bowl and no washing step is required. Also, in embodiments, the portable urination weight measurement device can be protected by a guard present on the container.

It is an advantage of embodiments of the present invention that they can be used in complete privacy, not giving any clue to anybody around the user that the invention has been used. Only the patient and the person who will analyse the results can know that the invention has been used. The portable urination weight measurement can be easily concealed in a handbag or a pocket; it does not require any washing after use and the container can be directly discarded in the toilet bowl.

It is an advantage of embodiments of the present invention that measurements may be recorded in real time. This permits for instance instant feedback to adjust drug (e.g. diuretics) and/or diet (e.g. salts and liquids) recommendations.

It is an advantage of embodiments of the present invention that urine volume and frequency can be easily recorded

It is an advantage of embodiments of the present invention that it may be ecological. The device and container do not need to be washed, hence limiting the use detergents; and the containers may be biodegradable.

It is an advantage of embodiments of the present invention that it may be easily re-used or shared between different users.

It is an advantage of embodiments of the present invention that it may be made available for a relatively low cost while providing very good performances.

It is an advantage of embodiments of the present invention that it may allow long term and secure data storage and reliable transfer of data between the user and the people analysing the data.

The above objective is accomplished by a method and device according to the present invention.

In a first aspect, the present invention relates to a portable urination weight measurement device comprising:
- a holding unit for releasably holding a container, and
- a handle comprising a weight measurement unit coupled to the holding unit in such a way as to allow the measure of a weight exerted on the holding unit.

In any embodiments, the weight measurement unit may comprise a weight sensor such as e.g. a strain gauge.

In any embodiments of the present invention, the weight measurement unit may comprise:
∘ a strain gauge, and
∘ an electronic circuit suitable for converting a strain measurement into a weight signal,
wherein the strain gauge is coupled to the holding unit in such a way as to allow the measurement of a strain caused by a weight exerted on the holding unit. For instance, the strain gauge may comprise a conductive grid pattern glued on an aluminium block. The conductive grid pattern may have leads for electrically connecting to the electronic circuit. In embodiments, the strain gauge may be equipped with a four-probe sensor. In embodiments, the strain gauge may be fixed to the handle case. In embodiments, the electronic circuit may comprise a strain gauge bridge and an analog to digital converter and the strain gauge bridge may be electrically connected to the strain gauge. The conversion of the strain measurement into a weight signal may be performed via this strain gauge bridge and this analog to digital converter. The electronic circuit typically further comprises a central processing unit CPU, a storage memory and a power management unit. The analog to digital converter may provide the analog to digital conversion of the sensed weight to the central processing unit (CPU). Preferably, the analog to digital converter uses a sampling speed of from 10 to 100 Hz. This is advantageous as it allows for movement and vibration handling. The electronic circuit is suitable for converting a strain measurement into a weight signal, wherein the strain gauge is coupled to the electronic circuit in such a way as to allow converting the strain measurement into a weight signal. In embodiments, the electronic circuit may be further adapted for determining a tilt angle of the portable urination weight measurement device so as to compensate for said tilt during conversion of the strain measurement into the weight signal and/or so as to inform a user of the tilt or of an information derived therefrom.

The strain gauge has a single axis of sensitivity and the measured weight is the component of the force exerted on the holding unit parallel to this axis of sensitivity. So, the tilt angle is the angle between the gravity vector, which is vertical, and this axis of sensitivity.

For determining the tilt angle, the electronic circuit may further comprise an accelerometer connected to the processor so as to permit the processor to compensate for the tilt angle during conversion of the strain into the weight signal and/or so as to inform a user of the tilt or of an information derived therefrom. The accelerometer may for instance be a 3-axis accelerometer. Since the angle between the 3-axis accelerometer and the axis of sensitivity of the strain gauge is both known and constant, if the position of the gravity vector relative to the 3-axis accelerometer is measured by the 3-axis accelerometer, the angle between the sensitivity axis and the gravity vector can be computed and the tilt can be computed.

As the strain gauge is only sensitive to the weight force parallel to its axis of sensitivity (which is typically perpendicular to the strain gauche plane), the tilt angle of the device is preferably known in order to compensate for it (in two directions). As it is known to the person skilled in the art, such a compensation can be achieved by implementing a suitable trigonometric formula either directly in the hardware of the CPU or in a software running therein. Such compensation provides sufficiently accurate results up to a tilt angle of about 15°. In an embodiment of the present invention, a user interface (see infra) may inform the user that the tilt angle is excessive (e.g. above 15°), e.g. when validating the measurement. If the user is informed of an excessive tilt before urination starts, the user can still correct the position of the device before urination and thereby improves the accuracy of the measurement. An example of information derived from the tilt is for instance instructions on how to better position the device before urination. If a urination occurs while the tilt was determined as being excessive, the person in charge of the data analysis will know that this measurement was faulty. Another example of information derived from the tilt is for instance the extent of the device's movements during measurement. If the device moves too much during measurement, the device may become unable to measure correctly the weight since the dynamic forces on the sensors may become too important for a precise measurement. The 3-axis accelerometer may be used to measure the average noise on the position of the gravity vector in order to inform the user that the amount of movement is too high for a precise measurement or in order to inform the person in charge of the data analysis that the measurement was faulty due to this excessive movement.

In any embodiments of the first aspect where the handle comprises an electronic circuit, the electronic circuit may be suitable for storing the measured weight signal or data derived therefrom. In embodiments, the electronic circuit may be further suitable for storing the time at which the weight signal is measured and, optionally, the duration of the measurement. The electronic circuit may comprise a memory connected to the CPU in such a way as to allow for storing the measured weight signal or data derived therefrom. The memory may be connected to the processor in such a way as to further allow for storing the time at which the weight signal is measured and, optionally, the duration of the measurement.

In any embodiments of the first aspect where the handle comprises an electronic circuit, the electronic circuit may be suitable for sending the measured (and typically stored) weight signal or data derived therefrom to a remote receiver. In embodiments, the electronic circuit may be further suitable for sending the measured (and typically stored) time at which the weight signal is measured and, optionally, the duration of the measurement, to a remote receiver. The electronic circuit may comprise an emitter connected to the CPU in such a way as to allow for sending the measured weight signal or data derived therefrom to a remote receiver. Emitter may be connected to the processor in such a way as to further allow for sending the time at which the weight signal is measured and, optionally, the duration of the measurement, to a remote receiver. The electronic circuit may comprise a receiver connected to the CPU in such a way as to allow for the configuration of the device from a distance or for sending information to the user from a remote emitter. The emitter and the receiver are preferably wireless. The electronic circuit may comprise a data exchange interface, such as a wireless interface, combining both signal emitting and receiving capacity. An example of such an interface is a Bluetooth wireless interface. Such an interface permits connection between the portable urination weight measurement device and a computer, smartphone or tablet for configuration and/or data exchange.

The electronic circuit may comprise a charging interface, such as a wireless charging interface, for charging the battery. For instance, the electronic circuit may comprise an induction receptor for charging the battery. This allows the battery to be charged by induction. Charging by induction requires an induction emitter, which does not need to form part of the portable urination weight measurement device. In embodiments, the induction emitter may be incorporated in a transport box. The transport box is suitable for transporting the portable urination weight measurement device. An aspect of the present invention relates to a kit of parts comprising the portable urination weight measurement device and the transport box. In embodiments, the transport box may comprise a power input port (e.g. a USB port) connected to the induction emitter in such a way as to allow it to be powered. Charging can be done by connecting a power cable to the transport box via the power input port. In embodiments, the charging may also be performed by connecting a power source to a USB interface forming part of the electronic circuit.

In any embodiment of the first aspect where the weight measurement unit comprises an electronic circuit, the electronic circuit may be further suitable for measuring the time at which the weight signal is measured. In embodiments, the electronic circuit may be further suitable for measuring the duration of the weight recording. For instance, the electronic circuit may comprise a real-time clock for recording the time at which the weight signal is measured and, optionally, the duration of the weight recording. In embodiments, the portable urination weight recorder may further comprise a coin cell backup battery holder electrically connected to the real-time clock. The coin cell backup battery holder may be suitable for holding a coin cell backup battery for keeping the real-time clock powered in case electrical power from another source is not available.

In any embodiments of the first aspect, the time duration measurement combined with the weight measurement, would offer further technical information such as urinal miction rythmics.

In any embodiments of the first aspect, the electronic circuit may further comprise a display coupled to the weight measurement unit in such a way as to allow displaying the measured weight.

In any embodiments of the first aspect, the electronic circuit may further comprise a mode selection switch, coupled to the weight measurement unit and to the display, for selecting between a weight measurement mode and one or more other modes. Amongst possible modes to be selected from, a user and an analysis mode can be present.

In any embodiments of the first aspect, the electronic circuit may comprise a mode validation switch, coupled to the weight measurement unit and to the display, for validating a selected mode.

In any embodiments of the first aspect, the electronic circuit may further comprise a power switch, coupled to the weight measurement unit, for turning the portable urination weight measurement device on and off. In embodiments, the power switch and the mode validation switch may be a same button, and the button may work as a power switch or a mode validation switch depending on the length of a pression on the button. For instance, a longer pression could turn the device on or off while a shorter pression could validate a selected mode.

When switches are present in any embodiments of the present invention, preferred are spring buttons (e.g. comprising an elastomeric material). However, any type of switches can be used in the present invention, including touch switches such as capacitance, resistance or piezo touch switches.

In embodiments where switches are present, the electronic circuit may comprise an electro-acoustic transducer, coupled to any of the previous switches, for audibly confirming the engagement of the switch.

In any embodiments of the first aspect, the electronic circuit may further comprise light sources such as LEDs. The CPU may be further connected so as to receive inputs from switches and so as to send outputs to one or more light sources.

When one or more of switches, a display, electro-acoustic transducers and light sources are present, they can be said to form the user interface. The user interface allows the guidance of the user in the whole process of recording the urination data and review of the recorded data by the user and/or the person who will analyse the data.

An example of procedure the device may follow making use of the user interface is:
1) Ask the user to attach the empty container to the holding unit and to indicate when the container is ready;
2) Acquire the weight of the empty container at at least one time;
3) Ask the user to start urinating;
4) Acquire the weight of the full container at at least one time;
5) Compute the weight of urine; and
6) Store the weight of urine in a memory.

Before step 1), one or more of the following steps may be added:
0) Ask the user if he wants to make a new urination or if he wants to access historical data.

In embodiments, the default option, ready to be selected by pression on the validation switch, could be the new urination.

Between step 1) and step 2), the following steps may be added:
1a) Ask the user to place the device horizontally (e.g. by asking the user to place the surface for resting with the toilet seat on the toilet seat);
1b) Ask the user to indicate when the device is horizontal;

During step 2), the following step may also be performed:
- Acquire of the tilt each time a weight is acquired and optionally compute an average tilt;
- Compute an average weight value (e.g. moving average);
- Compute the standard deviation on the weight during at least a time interval;
- Wait for the standard deviation on the weight to be below a set value before to perform step 3);
- Compute the standard deviation on the tilt during at least one time interval;
- Wait for the standard deviation on the tilt to be below a set value before to perform step 3);
- Wait for the tilt to be below a set value before to perform step 3);
- If the measured tare is not in a specified range, alerting the user that something is wrong with its device;
- If it applies, inform the user that the position of the device must be corrected;
- If it applies, inform the user that he moves too much;
- Display a level to assist the user in positioning the device;
- Ask the user to validate that the urination will start;

Between step 3) and step 4), the following step may also be performed: 3a) record continuously the weight and the angular position for further analysis.

During step 4), the following step may be performed:
- Filter the acquired data to take into account liquid waves present in the urine containing container.

During step 5), the following step may be performed:
- storing the whole curve for further analysis by the person who will analyse the data;
- transform the weight into a volume;

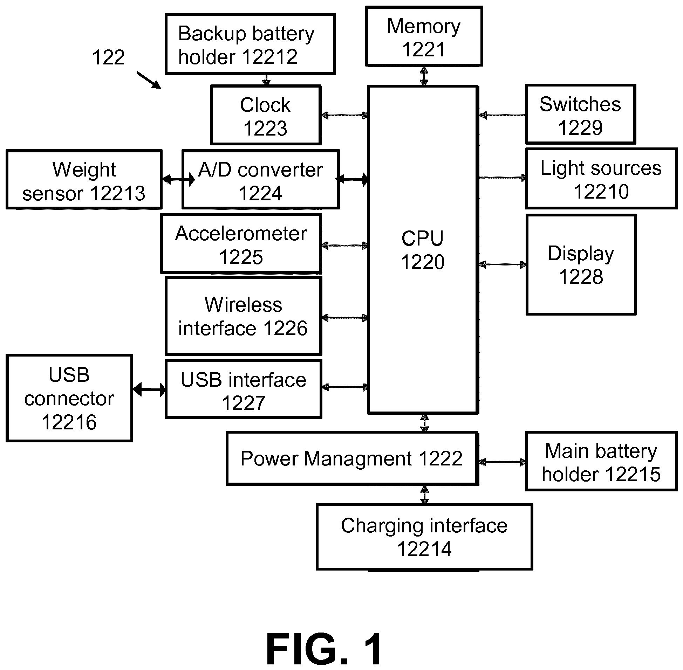

The above-described procedure embodiments of the present invention, such as the procedures followed making use of the user interface, may be implemented in an electronic circuit such as shown in Fig. 1. Fig. 1 shows one configuration of a processing system 122 that includes at least one programmable processor 1220 coupled to a memory subsystem 1221 that includes at least one form of memory, e.g., RAM, ROM, and so forth. Thus, one or more aspects of the present invention, and in particular the procedures followed by the user interface, can be implemented in digital electronic circuitry, in software, or in combinations of them. The processing system 122 may include a storage subsystem (USB interface) that has at least one input port (e.g. USB connector 12216). In some implementations, a display system (1228), switches (1229), and light sources (12210) and/or electro-acoustic transducers may be included as part of a user interface subsystem to provide for a user to receive and manually input information. More elements such as network connections, interfaces (e.g. wireless interface 1226) to various devices, and so forth, may be included. The various elements of the processing system 122 may be coupled in various ways. The memory of the memory subsystem 1221 may at some time hold part or all of a set of instructions that when executed on the processing system 122 implement the steps of method embodiments described herein. An embodiment of the present invention is a processing system (an integrated circuit) that includes the instructions to implement aspects of the methods for determining a weight or volume of a urination.

The present invention also includes a computer program or computer program product which provides the functionality of any of the methods according to the present invention when executed on a computing device. Such computer program product can be tangibly embodied in a carrier medium carrying machine-readable code for execution by the CPU. The present invention thus also relates to a carrier medium carrying a computer program product that, when executed on computing means, provides instructions for executing any of the methods as described above. The term *"carrier medium"* refers to any medium that participates in providing instructions to a processor for execution. Such a medium may take many forms, including but not limited to, non-volatile media, and transmission media. Non-volatile media includes, for example, optical or magnetic disks, such as a storage device which is part of mass storage. Common forms of computer readable media include, a CD-ROM, a DVD, a flexible disk or floppy disk, a memory key, a tape, a memory chip or cartridge or any other medium from which a CPU can read. Various forms of CPU readable media may be involved in carrying one or more sequences of one or more instructions to a processor for execution. The computer program or computer program product can be carried on an electrical carrier signal. The computer program product can also be transmitted via a carrier wave in a network, such as a LAN, a WAN or the Internet. Transmission media can take the form of acoustic or light waves, such as those generated during radio wave and infrared data communications. Transmission media include coaxial cables, copper wire and fibre optics, including the wires that comprise a bus within a computer.

In any embodiment of the present invention, the handle may comprise a protective case, which is typically water-tight and water-proof. This is advantageous since the device is meant to be used in presence of a urine stream. It typically comprises a bottom part and an upper part. The upper part may have an opening for exposing a display comprised in an electronic circuit. The upper part may have openings for exposing switches or may have depressible portions for enabling activation of switches comprised in an electronic circuit. The bottom part may enclose a battery holder or directly a battery without a holder. A battery may be included in the battery holder. The type of battery is not critical but a lithium polymer battery is advantageous due to its very good power density.

The bottom part of the case may have an opening at an extremity thereof for permitting the holding unit to penetrate the case in which it is attached to the weight measurement unit, e.g. via a strain gauge. The opening is preferably provided with a gasket allowing the case to remain water-tight while allowing the holding unit to displace itself vertically as a result of the weight exerted thereon. The height of the opening serves as a stop to restrain excessive movement of the holding unit and to thereby prevent damaging the weight measurement unit. The gasket is typically made of an elastomeric material.

In a preferred embodiment, the present invention may relate to a portable urination weight measurement device comprising:
- a holding unit for releasably holding a container, and
- a handle comprising a weight measurement unit coupled to the holding unit in such a way as to allow the measure of a weight exerted on the holding unit, the weight measurement unit comprising:
   ∘ a strain gauge, and
   ∘ an electronic circuit suitable for converting a strain measurement into a weight signal and further adapted for determining a tilt angle of the portable urination weight measurement device so as to compensate for said tilt during conversion of the strain measurement into the weight signal and/or so as to inform a user of the tilt or of an information derived therefrom,
wherein the strain gauge is coupled to the holding unit in such a way as to allow the measurement of a strain caused by a weight exerted on the holding unit and is coupled to the electronic circuit in such a way as to allow converting the strain measurement into a weight signal.

Preferably, the case may be designed so that any over pressure on the strain gauge is prevented by a stop on the path of displacement of the holding unit. This would insure the durability of the strain gauge.

The holding unit may be any means for holding a container such as e.g. a hook or a jaw. However, the holding unit preferably comprises a jaw as it more easily allows the mechanical release of the container without necessitating the user to touch the urine-filled container.

In any embodiments of the first aspect, the handle may comprise a release mechanism coupled to the holding unit in such a way as to allow the release of the container. This is advantageous as it does not necessitate manipulation of the urine-filled container to release it. In embodiments, the release mechanism may comprise a switch for releasing the container. In embodiments, the switch may be at the back of the handle, i.e. at the extremity away from the holding unit. In embodiments, the switch may act via a spring. In embodiments, the switch may be a pressure button. In embodiments, the release mechanism may comprise:
a. the pressure button accessible by a user and suitable for pushing on a rod,
b. the rod,
c. a rocker mechanism, and
d. an implement,
wherein the rod is contacting the pressure button,
wherein the rocker mechanism is coupled to the rod and to the implement in such a way that a forward motion of the rod translates into a backward motion of the implement,
wherein the implement (e.g. a fork) is coupled to the holding unit in such a way as to control its opening. This can for instance be achieved by the implement engaging on one hand with the rocker mechanism and on another hand with indentations in the holding unit, with pressure on the holding unit maintaining it close and release of the pressure opening it and liberating the container.

When the implement is a fork, the fork is coupled to the holding unit in such a way as to control its opening. This can for instance be achieved by the extremity of the fork handle engaging with the rocker mechanism and the extremities of the fork teethes engaging with indentations in the holding unit, with pressure on the holding unit maintaining it close and release of the pressure opening it and liberating the container.

In embodiments where a rocker mechanism is present, it may comprise:
a. A rocker lever axis,
b. A self-pressure spring,
c. A rocker control lever,
wherein the self-pressure spring and the rocker control lever are engaged on the rocker lever axis and wherein the rocker control lever is engaged with the rod and with implement.

In any embodiments of the first aspect, the handle may comprise a first surface for resting on a toilet seat and wherein the holding unit is suitable for extending downward when the surface for resting on a toilet seat is resting on a toilet seat. When the holding unit comprises a jaw, it is the opening of the jaw which is extending downward when the surface for resting on a toilet seat is resting on a toilet seat. In embodiments, the part of the jaw closest to the back of the device may be at an angle of from 91 to 120°, preferably from 95 to 110° with respect to the surface for resting on a toilet seat. 90° tends to be insufficient in some cases for the container not to touch the walls of the toilet bowl. An angle close to 90° is however best for the release of the container after use. The angle ranges proposed herein are therefore good compromises. In embodiments, the surface for resting on a toilet seat may comprise means for hindering movements of the surface when in contact with the toilet seat. The presence of these means help maintaining the device still during measurements, which increases accuracy. In embodiments, the means for hindering movements are means for causing friction between the surface and the toilet seat. For instance, these means may consist in a piece of rubbery (i.e. elastomeric) material such as e.g. polyurethane. The piece of rubbery material may for instance have an arc shape. For instance, the end points of the arc may be oriented toward the front of the handle, i.e. toward the holding unit.

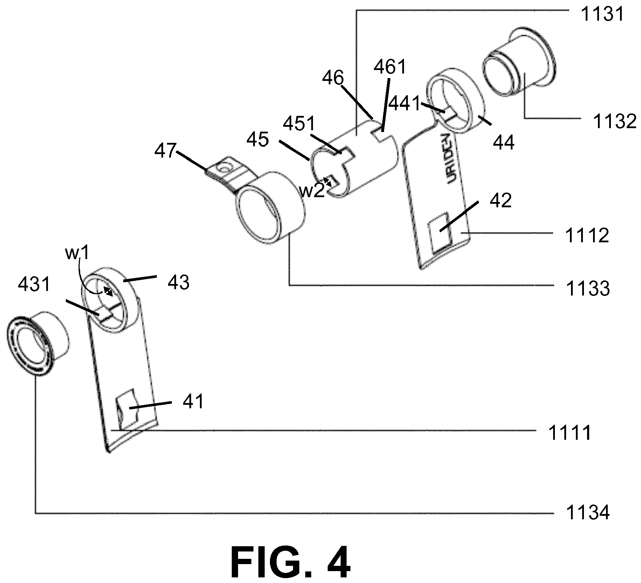

In any embodiments of the first aspect, the holding unit may comprise a jaw comprising two opposing parts that close for holding the container. In embodiments, each opposing part may comprise a flat plate. Examples of lengths for the flat plates are from 4 to 8, preferably 5 to 7 cm. Examples of width for the flat plates are from 1.5 to 4 cm, preferably 2 to 3 cm. Examples of thicknesses for the flat plates (not counting the protrusion) are from 0.5 to 3 mm, preferably 1 to 2 mm. In embodiments, the first opposing part may comprise a concave entity extending perpendicularly to a surface thereof facing the second opposing part, and the second opposing part may comprise a convex entity extending perpendicularly to a surface thereof facing the first opposing part, the convex entity fitting with the concave entity. Instead of a convex entity, a hole fitting with the concave entity can be provided. The combination of the convex entity and the concave entity forms a locking system for preventing the container from falling. This is illustrated by items 41 and 42 in Fig. 4. In embodiments where the holding unit comprises a jaw, the release mechanism may release the container by opening the jaw by a few degrees. For instance, the jaw may be designed in such a way that its two opposing constituting parts are separated by a few degrees (e.g. 1 to 5 degrees) in absence of external forces and a spring in the release mechanism may exert a constant external force on the parts of the jaw that keeps it close as long as a switch of the release mechanism is not activated. Once the switch of the release mechanism is activated, the external force exerted by the release mechanism is alleviated and the jaw takes its resting open position.

In any embodiments of the first aspect, the holding unit may be rotatably attached to the handle in such a way that the portable device can be folded by covering a second surface of the handle with a surface of the holding unit. This is advantageous as it permits the device to take a minimum amount of space when not in use. This makes the device less bulky and hence easier to transport. Also, it protects the holding unit from damages. For instance, making the holding unit rotatably attached to the handle can be achieved via a hinge holding the handle and the holding unit together. In embodiments, the hinge may belong to the holding unit. In embodiments, the hinge may comprise:
a. A male fastener,
b. A female fastener for mating with the male fastener,
c. A ring attached to the first opposing part of the jaw, the ring having at least one protrusion on its inner side, the protrusion having a first width w1, the first width w1 being measured along the curvature of the ring,
d. A ring attached to the second opposing part of the jaw, the ring having at least one protrusion on its inner side, the protrusion having the first width w1,
e. A central control ring comprising two ends and at least one cut at each end, the cut having a second width w2, larger or equal to the first width w1, measured along the curvature of the central control ring, the central control ring being of a diameter such that it can engage in both rings attached to the parts of the jaw in such a way that the cuts are penetrated by the protrusions,
f. A weighting control ring bearing an arm projecting outward, the weighting control ring having a diameter such that it can be penetrated by the central control ring,
wherein the male fastener and the female fastener, once mated, form an axis of rotation around which the ring bearing the first part of the jaw, the central control ring, and the ring bearing the second part of the jaw can rotate,
wherein the arm is attached to the weight measurement unit.

The widths w2 and w1 are preferably such that they friction fit. w2 is preferably equal to w1 or larger by less than 1 %, preferably less than 0.2% such as from 0.03% ot 0.2% or 0.03% to 0.1 %.

When indentations are present in the holding unit to permit its engaging with an implement as discussed above, these indentations are preferably on the rings attached to the first and second part of the jaw.

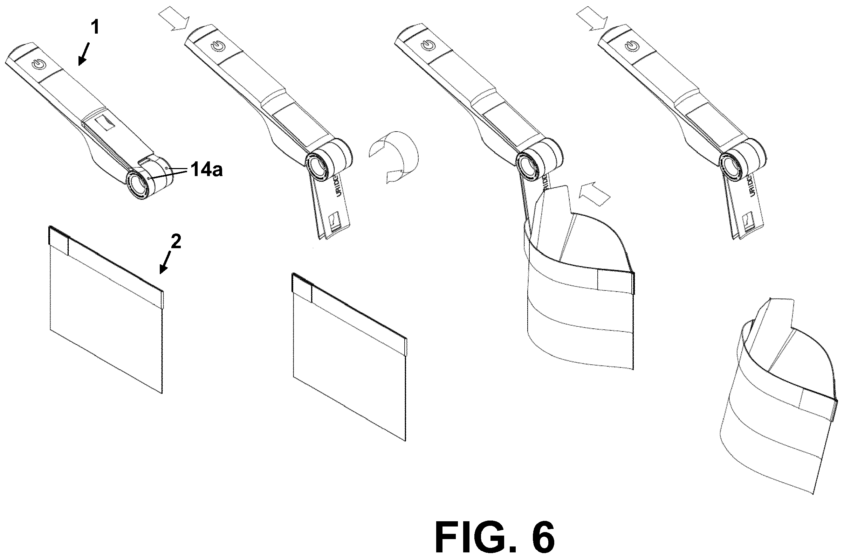

In embodiments, the ring attached to a first part of the jaw and the ring attached to the second part of the jaw may each have an indentation for engaging with the implement of the release mechanism. These indentations may be positioned on the ring in such a way that once engaged with the implement, the jaw is in a position where the device is ready for use, i.e. with the jaw extending downward. Typically, this translates into having the indentations at positions on the rings attached to the first and second parts of the jaw which are visible to an observer looking at the folded device from above. This is illustrated in the first representation from the left in Fig. 6 where these indentations (14a) are visible.

Further indentations may also be present on the holding unit, and in particular on the rings attached to the first and second parts of the jaw, in order to engage with the implement when the device is folded (with a second surface of the handle covering a surface of the holding unit) and ready for transport.

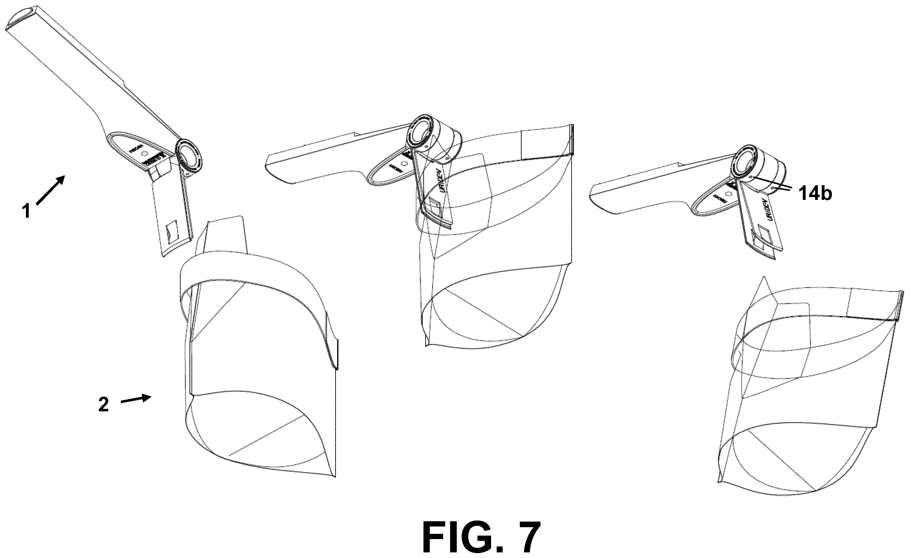

Typically, this translates into having the indentations at positions on the rings attached to the first and second parts of the jaw which are visible to an observer looking at the unfolded device from below. This is illustrated in Fig. 7 where these indentations (14b) are visible.

In embodiments, the strain gauge may be connected to the arm of the weighting control ring. This permits the strain gauge to sense the weight exerted on the arm by the action of the container on the jaw attached thereto.

In embodiments, the weighting control ring bearing an arm may be in aluminium.

When, as mentioned before, the bottom part of the case has an opening at an extremity thereof for permitting the holding unit to penetrate the case, and when the hinge comprises a weighting control ring bearing an arm, the part of the holding unit which may be attached to the weight measurement unit may be the arm. In that case, the opening is preferably provided with a gasket allowing the case to remain water-tight while allowing the arm to displace itself vertically as a result of the weight exerted thereon. Also, the height of the opening serves as a stop to restraint excessive movement of the arm and to thereby prevent damaging the weight measurement unit.

In any embodiments of the present invention, the weight measurement unit may comprise a stop for limiting the possible movements of the holding unit. In embodiments where the holding unit comprises an arm, the weight measurement unit may comprise a stop for limiting the possible movements of the arm.

In a second aspect, the present invention relates to a toilet flushable container having:
- a rim delimiting its opening and having a fold at a front side of the opening and a fold at a back side of the opening, opposite to the fold at the front side, each fold forming at rest an angle of less than 20°, and
- a collar secured to the rim, the collar comprising at least one fold at the front side of the opening forming at rest an angle of less than 20°, the collar being of a rigidity such that increasing the angle of the at least one fold to 180° increases the angle of the rim fold at the back of the opening from its rest angle of less than 20° to an angle of from 50 to 90°.

In embodiments, increasing the angle of the at least one fold to 180° does not introduce further folds in the collar.

In embodiments, the collar may be a strip secured to the outside of a sidewall of the container via at least two attachment points and wherein a distance along the strip between two of the at least two attachment points is larger than a corresponding distance along the sidewall of the container along said two of the at least two attachment points, thereby forming a gap between the strip and the sidewall of the container. In embodiments, the collar may be a ring around the sidewall of the container. Preferably, the collar may run around the rim of the container, i.e. around the rim of the sidewall of the container. In embodiments, the collar may be secured to the rim of the container.

The rim folds and the at least one collar fold maintain the container relatively closed in absence of pinching of the collar fold. Preferably, each fold may form at rest an angle of less than 15°, more preferably less than 10°, yet more preferably less than 5°.

The shape of the opening when the collar makes an angle of 180° at the front side and when the rim makes an angle of from 50 to 90° at the back side is particularly well suited for fitting between the legs of the user.

Increasing the angle of the at least one collar fold to 180° opens the container. Once open in that way, the container is ready for use. The collar opening thereby obtained has a pointy side and an opposite flat side. Opening the fold to 180° is typically done by pinching a collar fold. The container is particularly well adapted for use together with a portable urination weight measurement device which holding unit comprises a jaw suitable for pinching a fold and thereby opening the collar to 180°. A jaw having opposing parts comprising flat plates is particularly adequate for this purpose.

The rigidity of the collar is preferably such that it prevents torsion thereof when the container is filled with urine. In embodiments, the rigidity of the collar may be such that it maintains the top of the collar in a single plane even when the container is filled with urine.

As used herein and unless provided otherwise, the term *"toilet flushable"* when applied to a container refers to a container which, when full of urine, can be flushed in a typical toilet without blocking it. This can for instance be achieved by having the container made of flexible materials. Flexible materials can adapt to the shape of the toilet trap and waste pipe. For this purpose and in such embodiments, the collar is preferably made of a material assuring enough rigidity for it to prevent torsion thereof when the container is filled with urine but flexible enough for it to be flushable. A preferred characteristic of toilet flushable container is bio-degradability. Indeed, in many countries, bio-degradability is a legal requirement for anything meant to be flushed in a toilet. Toilet flushability can also be achieved by making the container water-soluble.

In any embodiment of the second aspect, the toilet flushable container may be water-soluble and suitable for containing fresh human urine for 40 seconds without leaking. Water solubility is advantageous as it either enables or improves toilet flushability. Suitable materials for forming the container include polyethylene glycol; alkylcellulose derivatives such as hydroxymethylcellulose, hydroxyethylcellulose, and carboxymethylcellulose; polyvinylacetate, polyvinylalcohol, carboxylated acrylic polymers, hydrosoluble polyurethane, copolymers thereof, derivatives thereof and combination of such materials. Preferably the material may be polyvinylalcohol partially or fully hydrolysed, such as 80-99% hydrolysed. Advantageously, the container may be formed of a plurality of hydrosoluble polymer sheets having the same or different solubilities. Advantageously, the container may be composed of two layers of polyvinylalcohol having the same or preferably different solubilities. Also usable are a combination of a soluble material and reinforcing fibres, preferably natural fibers. For instance, the container may be made of sodium carboxymethylcellulose that may be reinforced by natural fibers. Plasticizers may be added to the material to achieve the desired level of solubility.

In any embodiment of the second aspect, the toilet flushable container may have a capacity of from 600 ml to 1000 ml, preferably from 600 ml to 700 ml.

In any embodiment of the second aspect, the toilet flushable container may comprise means for attaching to a holding unit without penetration of the holding unit in the container. In embodiments, this means may be suitable for attaching the holding unit at the front side of the container. In an embodiment, this means may be the collar forming gap between the collar and the rim of the container. The holding unit can hold the collar while penetrating this gap, thereby not penetrating the container. The gap is preferably situated at one of the opposite sides of the collar opening. When the holding unit comprises a jaw, one of the opposite parts of the jaw may penetrate in the gap, thereby allowing the pinching of the fold upon closing of the jaw.

In any embodiment of the second aspect, the toilet flushable container may comprise a guard for protecting a holding unit from urine projections. In preferred embodiments, this guard may take the form of a wall at the front side of the container being higher than a wall at the opposite back side of the container. Preferably, the guard extends above the collar when the container is open and is foldable inside the container, below the collar, when the container is closed. This way, the guard can take minimal space when the container is closed and can be (automatically) deployed upon opening the container.

In a third aspect, the present invention relates to a kit of parts comprising:
a. A portable urination weight measurement device according to any embodiment of the first aspect, and
b. A toilet flushable container according to any embodiment of the second aspect.

Particular and preferred aspects of the invention are set out in the accompanying independent and dependent claims. Features from the dependent claims may be combined with features of the independent claims and with features of other dependent claims as appropriate and not merely as explicitly set out in the claims.

Although there has been constant improvement, change and evolution of devices in this field, the present concepts are believed to represent substantial new and novel improvements, including departures from prior practices, resulting in the provision of more efficient, stable and reliable devices of this nature.

The above and other characteristics, features and advantages of the present invention will become apparent from the following detailed description, taken in conjunction with the accompanying drawings, which illustrate, by way of example, the principles of the invention. This description is given for the sake of example only, without limiting the scope of the invention. The reference figures quoted below refer to the attached drawings.

### Brief description of the drawings

Fig. 1 is a block diagram illustrating a highly simplified form of an electronic circuit usable in the invention.
Fig. 2 is a schematic representation of a top, side and bottom view of a portable urination weight measurement device according to an embodiment of the present invention.
Fig. 3 is an exploded view showing parts of a portable urination weight measurement device according to an embodiment of the present invention.
Fig. 4 is an enlarged fragmentary view showing more detail of the hinge of the device of Fig. 3.
Fig. 5 are various schematic representations of a toilet flushable container in its open and closed state.
Fig. 6 and 7 show schematically sequences implementing a way embodiments of the present invention can be used.
Fig. 8 is a schematic representation of a side view of a portable urination weight measurement device according to an embodiment of the present invention.

In the different figures, the same reference signs refer to the same or analogous elements.

### Description of illustrative embodiments

The present invention will be described with respect to particular embodiments and with reference to certain drawings but the invention is not limited thereto but only by the claims. The drawings described are only schematic and are non-limiting. In the drawings, the size of some of the elements may be exaggerated and not drawn on scale for illustrative purposes. The dimensions and the relative dimensions do not correspond to actual reductions to practice of the invention.

Furthermore, the terms first, second, third and the like in the description and in the claims, are used for distinguishing between similar elements and not necessarily for describing a sequence, either temporally, spatially, in ranking or in any other manner. It is to be understood that the terms so used are interchangeable under appropriate circumstances and that the embodiments of the invention described herein are capable of operation in other sequences than described or illustrated herein.

Moreover, the terms top, bottom, over, under and the like in the description and the claims are used for descriptive purposes and not necessarily for describing relative positions. It is to be understood that the terms so used are interchangeable under appropriate circumstances and that the embodiments of the invention described herein are capable of operation in other orientations than described or illustrated herein.

It is to be noticed that the term "comprising", used in the claims, should not be interpreted as being restricted to the means listed thereafter; it does not exclude other elements or steps. It is thus to be interpreted as specifying the presence of the stated features, integers, steps or components as referred to, but does not preclude the presence or addition of one or more other features, integers, steps or components, or groups thereof. Thus, the scope of the expression "a device comprising means A and B" should not be limited to devices consisting only of components A and B. It means that with respect to the present invention, the only relevant components of the device are A and B.

Similarly, it is to be noticed that the term "coupled", also used in the claims, should not be interpreted as being restricted to direct connections only. The terms "coupled" and "connected", along with their derivatives, may be used. It should be understood that these terms are not intended as synonyms for each other. Thus, the scope of the expression "a device A coupled to a device B" should not be limited to devices or systems wherein an output of device A is directly connected to an input of device B. It means that there exists a path between an output of A and an input of B which may be a path including other devices or means. "Coupled" may mean that two or more elements are either in direct physical or electrical contact, or that two or more elements are not in direct contact with each other but yet still co-operate or interact with each other.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

Furthermore, some of the embodiments are described herein as a method or combination of elements of a method that can be implemented by a processor of a computer system or by other means of carrying out the function. Thus, a processor with the necessary instructions for carrying out such a method or element of a method forms a means for carrying out the method or element of a method. Furthermore, an element described herein of an apparatus embodiment is an example of a means for carrying out the function performed by the element for the purpose of carrying out the invention.

In the description provided herein, numerous specific details are set forth. However, it is understood that embodiments of the invention may be practiced without these specific details. In other instances, well-known methods, structures and techniques have not been shown in detail in order not to obscure an understanding of this description.

The following terms are provided solely to aid in the understanding of the invention.

The invention will now be described by a detailed description of several embodiments of the invention. It is clear that other embodiments of the invention can be configured according to the knowledge of persons skilled in the art without departing from the true spirit or technical teaching of the invention, the invention being limited only by the terms of the appended claims.

In Fig. 1, a diagram is shown summarizing the major parts of an electronic circuit (122) suitable for use in the first and the third aspect of the present invention. Double arrows indicate connections between parts with communication being able to go both ways. Single arrows indicated connections between parts with communication going one way. Central to the electronic circuit (122) is the central processing unit (CPU) (1220) which is connected so as to allow a two-way communication with a measurement storage memory (1221), a power management unit (1222), a real-time clock (1223), an analog to digital converter (1224), a three-axis accelerometer (1225), a Bluetooth wireless interface (1226), a USB interface (1227), and a LCD display (1228). The two-way communication between the CPU 1220 and, on one hand, the weight sensor (12213) via the analog to digital convertor (1224) and, on the other hand, the three-axis accelerometer (1225) permits the CPU (1220) to determine the tilt angle of the portable urination weight measurement device (1) so as to compensate for said tilt during conversion of the strain measurement into the weight signal and/or so as to inform a user of the tilt or of an information derived thereof. The two-way connection between the real time clock (1223) and the CPU (1220) permits to measure the time at which the weight signal is measured and the duration of the measurement. The Bluetooth wireless interface (1226) is an emitter (1226) connected to the CPU (1220) in such a way as to allow for sending the measured weight signal or data derived therefrom, the time at which the weight signal is measured and, optionally, the duration of the measurement, to a remote receiver. The central processing unit (CPU) is further connected so as to receive inputs from switches (1229) and so as to send outputs to one or more light sources (12210) (e.g. LEDs). The real-time clock (12211) is connected to a backup coin cell holder (12212) in such a way as to be able to be powered by a backup coin cell. The analog to digital converter is connected so as to allow a two-ways communication with a weight sensor (12213) (e.g. a strain gauge bridge). The USB interface can for instance be connectable so as to allow a two-ways communication with a micro-USB connector (12216). The power management unit (1222) is connected so as to allow a two-way communication with a wireless charging interface (12214). The power management unit (1222) is further connected so as to allow a two-way communication with a main battery holder (12215) (or directly with a main battery (13) if the battery is directly integrated without holder).

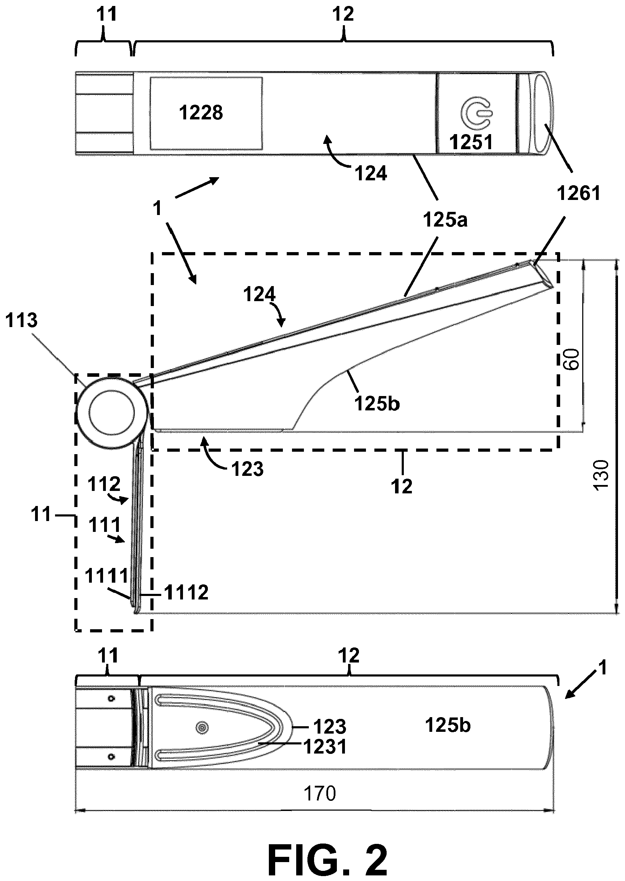

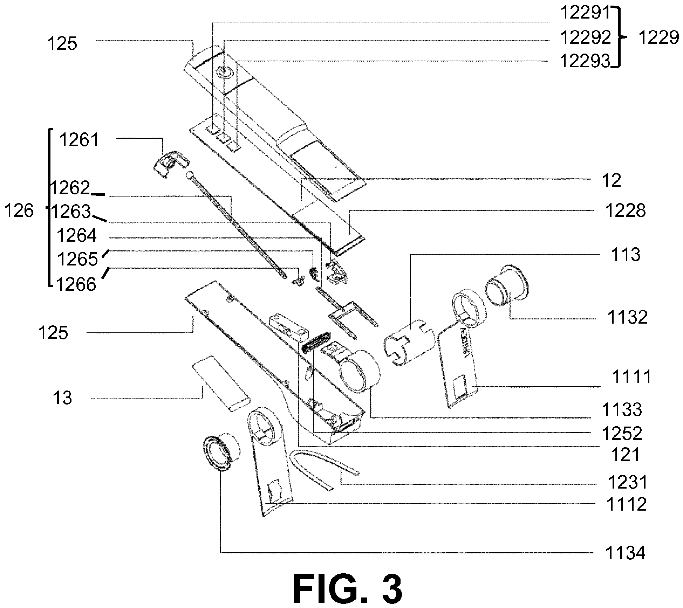

Fig.2 shows a top view (at the top of the figure), a side view (in the middle of the figure), and a bottom view (at the bottom of the figure) of an embodiment of the portable urination weight measurement device (1) of the first aspect. In the top, side and bottom views, a holding unit (11) and a handle (12) are visible. The handle (12) comprises a protective case (125) of which an upper part (125a) is visible in the top and side views, and a bottom part (125b) is visible in the side and bottom views. In the top view, the upper part (125a) of the protective case (125) is visible as having an opening exposing a display (1228) and as having a button (1251) covering switches (1229) (visible in Fig. 3). The depicted button (1251) is a spring button comprising an elastomeric material. In the top and side views, a switch (1261) for releasing a container is visible. This switch (1261) is at the back of the handle (12), i.e. at the extremity away from the holding unit (11). The depicted switch (1261) is a pressure button (1261). In the side view, the holding unit (11) is depicted as extending downward when the surface (123) for resting on a toilet seat is horizontal, as it would be if resting on a toilet seat. The part of the holding unit (11) extending downward is a jaw (111) comprising two opposing parts (1111, 1112) depicted at rest in a closed position. Each opposing part (1111, 1112) comprises a flat plate. The holding unit (11) is rotatably attached to the handle (12) in such a way that the portable device (1) can be folded by covering a second surface (124) of the handle (12) with a surface (112) of the holding unit. This is achieved via a hinge (113), belonging to the holding unit (11), and holding the handle (12) and the holding unit (11) together. In the embodiment depicted, the hinge (113) has a diameter of 25 mm. Details of the hinge (113) are given in Fig. 3. The height of the handle (12) from the bottom of the second part (125b) to the top of the first part (125a) is in the depicted embodiment 60 cm. The height of the device (1) when the jaw (111) is deployed so as to extend downward at 90° with respect to the surface (123) is 130 mm in the depicted embodiment. In the side and bottom views, the handle is shown to comprise a first surface (123) for resting on a toilet seat. In the bottom view, the surface (123) for resting on a toilet seat is depicted as comprising means (1231) for hindering movements of the surface (123) when in contact with the toilet seat. The depicted means (1231) are means (1231) for causing friction between the surface (123) and the toilet seat. The depicted means (1231) is a piece of elastomeric polyurethane having an arc shape which end points are oriented toward the front of the handle (12), i.e. toward the holding unit (11). The length of the device (1) in folded position corresponds to the length of the handle (11) added to the diameter of the hinge (113) totalling 170 mm in the present embodiment.

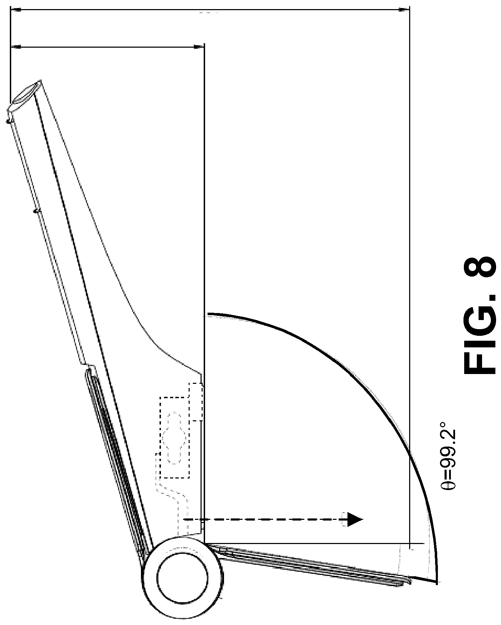

Fig. 8 is again a side view of a portable urination weight measurement device (1) according to an embodiment of the present invention, but this time an angle θ larger than 90° is depicted preventing the container (2), once attached to the holding unit (11), from touching the toilet bowl.

Fig. 3 shows an exploded view of a device (1) according to an embodiment of the present invention. Visible are elements (121, 122, 1228, 12291, 12292, 12293, 1231, 125a, 125b, 1252, 1261, 1262, 1263, 1264, 1265, and 1266) forming part of the handle (12), elements (1111, 1112, 1131, 1132, 1133, and 1b134) forming part of the holding unit (11), and a battery (13). The handle (12) comprises of a protective case (125) formed of an upper part (125a) and a bottom part (125b).

The bottom part (125b) encloses a battery holder. A battery (13) may be included in the battery holder. Also depicted is a piece of elastomeric polyurethane (1231) having an arc shape which end points are oriented toward the front of the handle (12), i.e. toward the holding unit (11).

The bottom part (125b) has an opening at an extremity thereof for permitting the holding unit (11) to penetrate the case (125) in which it is attached to the weight measurement unit (121, 122), e.g. via a strain gauge (121). The part of the holding unit (11) which penetrates the case (125) is the arm of the weighting control ring (1133). The opening is provided with a gasket (1252) allowing the case (125) to remain water-tight while allowing the holding unit (11) to displace itself vertically as a result of the weight exerted thereon. The height of the opening serves as a stop to restrain excessive movement of the holding unit (11) and to thereby prevent damaging the weight measurement unit (121, 122).

The weight measurement unit may comprise a strain gauge (121) and an electronic circuit (122) suitable for converting a strain measurement into a weight signal. The strain gauge is coupled to the holding unit (11) in such a way as to allow the measurement of a strain caused by a weight exerted on the holding unit (11). The strain gauge (121) is fixed to the bottom part (125b) of the handle case (125). The strain gauge (121) is coupled to the electronic circuit (122) in such a way as to allow converting the strain measurement into a weight signal.

The handle (12) further comprises a release mechanism (126) coupled to the holding unit (11) in such a way as to allow the release of the container (2). The release mechanism (126) comprises a pressure button (1261) for releasing the container (2). The pressure button (1261) is at the back of the handle (125), i.e. at the extremity away from the holding unit (11), and is accessible by a user. The pressure button (1261) acts via a spring (1265). The pressure button (1261) is connected to a rod (1262) so that a pressure on the pressure button (1261) translates into a movement of the rod (1262) along its length. The release mechanism further comprises a rocker mechanism (1263, 1265, 1266) and a fork (1264). The rocker mechanism (1263, 1265, 1266) is coupled to the rod (1262) and to the fork (1264) in such a way that a forward motion of the rod (1262) translates into a backward motion of the fork (1264). The rocker mechanism comprises a rocker lever axis (1263), a self-pressure spring (1265) and a rocker control lever (1266). The self-pressure spring (1265) and the rocker control lever (1266) are engaged on the rocker lever axis (1263). The rocker control lever (1266) is engaged with the rod (1262) and with fork (1264). The fork (1264) is coupled to the holding unit (11) in such a way as to control its opening.

Also visible in Fig. 3 are various parts composing the holding unit (11). The two opposing parts (1111 and 1112) of the jaw (111) that close for holding the container (2) are visible. Each opposing part (1111 and 1112) comprises a flat plate and a ring. The first opposing part (1111) comprises a concave entity extending perpendicularly to a surface thereof facing the second opposing part (1112), and the second opposing part (1112) comprises a hole facing the first opposing part (1111), the hole fitting with the concave entity. The combination of the hole and the concave entity forms a locking system for preventing the container (2) from falling. The release mechanism releases the container by opening the jaw by a few degrees.

Visible in Fig. 3 and Fig. 4 are parts of a hinge holding the handle (12) and the holding unit (11) together. The hinge belongs to the holding unit (11) and comprises a male fastener (1132); a female fastener (1134) for mating with the male fastener (1132); a ring (43) belonging to the first part (1111) of the jaw, the ring (43) having at least one protrusion (431) on its inner side, the protrusion (431) having a first width w1, the first width w1 being measured along the curvature of the ring (43); a ring (44) belonging to a second part (1112) of the jaw, the ring having at least one protrusion (441) on its inner side, the protrusion (441) having the first width w1; a central control ring (1131) comprising two ends (45, 46) and at least one indentation (451, 461) at each end (45, 46), the indentation having a second width w2, slightly larger than the first width w1, measured along the curvature of the central control ring (1131), the central control ring (1131) being of a diameter such that it can engage in both rings (43, 44) attached to the parts (1111, 1112) of the jaw in such a way that the indentations (451, 461) are penetrated by the protrusions (431,441), a weighting control ring (1133) bearing an arm (47) projecting outward, the weighting control ring (1133) having a diameter such that it can be penetrated by the central control ring (1131),

The male fastener (1132) and the female fastener (1134), once mated, form an axis of rotation around which the ring (43) belonging to the first part (1111) of the jaw, the central control ring (1131), and the ring (44) belonging to the second part (1112) of the jaw can rotate. The arm (47) is attached to the weight measurement unit via attachment to the strain gauge (121).

We now refer back to Fig. 3 only. The electronic circuit (122) is shown to comprise a display (1228) and switches (1229). The display (1228) is coupled to the weight measurement unit in such a way as to allow displaying the measured weight. Three switches (1229) are shown. Switch (12291) is an upper scroll control (12291), switch (12293) is a lower scroll control (12293), and switch (12293) is a power and mode validation switch (12292). Switches (12291) and (12293) are mode selection switches. Acting on these switches permits to scroll up and down in a menu, thereby highlighting and therefore selecting modes. The power and mode validation switch (12292) is coupled to the weight measurement unit and the display (1228) for validating a selection between a weight measurement mode and one or more other modes. It can work as a power switch or a mode validation switch depending on the length of a pression on the switch (12292). The three switches (12291, 12292, and 12293) are accessible by pression on different zones of the button (1229).

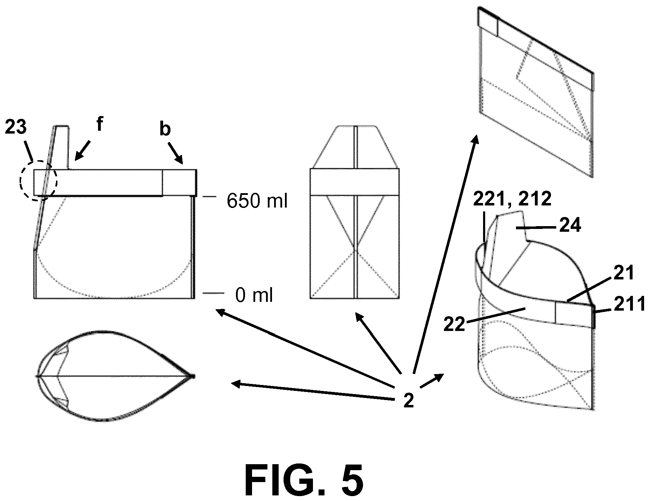

Fig. 5 shows five different views of a toilet flushable container (2). It has a rim (21) delimiting its opening and it has a fold (212) at a front side (f) of the opening and a fold (211) at a back side (b) of the opening, opposite to the fold (212) at the front side (f), each fold (211, 212) forming at rest an angle of less than 20°. It has a collar (22) secured to the rim (21), the collar (22) comprises at least one fold (221) at the front side of the opening forming at rest an angle of less than 20° (see view of the folded container at the top right of the figure), the collar (22) is of a rigidity such that increasing the angle of the at least one fold (221) to 180° increases the angle of the rim fold (211) at the back (b) of the opening from less than 20° to from 50 to 90° (see view of the open container (2) at the bottom left of the figure where the open container (2) is seen from above.

The collar (22) is a strip (22) secured to the outside of a sidewall of the container (2) and in particular to the rim (21) thereof, via at least two attachment points and the distance along the strip between two of the at least two attachment points is larger than a corresponding distance along the sidewall (or rim thereof) of the container (2) along said two of the at least two attachment points, thereby forming a gap (23) between the strip (22) and the sidewall of the container (2). The gap (23) is at the front side (f) of the container (2) and serves as a means (23) for attaching to a holding unit (11) without penetration of the holding unit (11) in the container (2). The toilet flushable container (2) comprises a guard (24) for protecting a holding unit (11) from urine projections.

The depicted container (2) has a capacity of 650 ml.

Fig. 6 and 7 show how the portable urination weight measurement device (1) and the toilet flushable container (2) can be used in combination. In the first representation from the left of Fig. 6, the portable urination measurement device (1) and the container (2) are both folded close. In the second representation of Fig. 6, the portable urination measurement device (1) is unfolded open as indicated by the curled arrow. The arrow pointing to the button (1261) indicates that pressure thereon open the jaw of the holding unit (11). In the third representation from the left of Fig. 6, one of the opposite parts (1111) of the jaw is depicted penetrating the gap (23). The arrow indicates that the jaw is closed on the fold (221) of the collar (22), thereby pinching it and opening the container (2). The device is then ready to collect and weight urine. In the last representation of Fig. 6, the arrow indicates that the button (1261) is depressed, thereby opening the jaw and releasing the container and the urine it contains. The container can be directly released in the toilet bowl because the container is flushable.

Fig. 7 shows on the left the opened device (1) and an open container (2). The container is shown open so that its constituting parts are more clearly visible but the container (2) does normally not open before the jaw pinches a fold in the collar thereof. At the center of Fig. 7, the device (1) and the container (2) are shown ready to collect urine. The container is depicted as transparent to facilitate the visualisation of how the device (1) grips the container (2). At the right of Fig. 7, the container (2) is released.

Hereafter is an example of procedure the device may follow making use of the user interface:
After the device is powered on using the power switch:
   1 Ask the user if it wants to make a new urination or if he wants to access historical data

The default option, ready to be selected by pression on the validation switch, could be the new urination.
2 Ask the user to fix the container and to press the selection validation switch to confirm the container is ready;
3 Ask the user to press a button when the device is almost horizontal (e.g. by asking the user to place the surface for resting with the toilet seat on the toilet seat) in order to measure the tare of the device before urinating;

During tare measurement, the device may start to make acquisitions of the weight (A/D conversion of the weight sensor) and, at the same time, make acquisitions of the 3-axis acceleration. Using these data, the CPU (e.g. via its embedded software) can for instance:
a. Compute an average weight value (moving average);
b. Compute the *"noise"* on the weight (e.g. standard deviation) in order to have a metric about the stability of the measure (e.g. if the user is moving too much);
c. Wait for this *"noise"* to be small enough, meaning the position is stable;
d. Compute an average angular position of the gravity vector using the 3-axis accelerometer;
e. Compute the *"noise"* on the angular position of the gravity vector (e.g. standard deviation) in order to have a metric about the stability of the measure (e.g. if the user is moving too much);
f. If the weight is stable enough AND if the angular position is stable enough AND if the angular position of the gravity vector is close enough from zero (meaning the sensor is horizontal - less than 15 degrees), the computed average weight can be selected as the reference tare with a trigonometric compensation on the horizontality using the computed average angular position of the gravity vector;
g. If not, a message may be shown to the user telling that either he's moving too much or that the horizontality is not good enough. In this latter case, a level logo may be displayed in real-time showing a *"bubble"* not in the center of a circle. The tare measurement process may be repeated until a good value is obtained or the user cancels the diuresis action;
h. When the tare is measured, the user may be asked to validate that the urination will start by activating a switch;
i. If the measured tare is not in a specified range (known weight of the clip and disposable part), an alert message can be displayed telling the user that something is wrong with its device (strain gage damaged, bad usage of the device during tare measurement, ...)

∘ Tell the user the tilt angle is too high if it applies;
∘ Tell the user the movements are too high if it applies;
∘ Tell the user he/she can start urinating;

During the urination, the CPU doesn't have to make any specific computation or acquisition for a simple measurement, but in embodiments, it may record continuously the weight and the angular position for further analysis. A simple logo tells the user to urinate and to press a button when urination is over.

When the diuresis is over and the user validates it with the button, the measurement process of the total weight (tare + diuresis) can start similarly to the tare measurement with the combined data from the weight sensor and 3-axis accelerometer. A difference is that since urine is present in the disposable part, liquid waves can occur because of the movements of the user's hand. These waves would then be present in the measured signals and a proper adaptive filtering may be done by the CPU in order to have a good balance between the precision of the measure and the necessary "*quite*" time of the liquid. The CPU may therefore be adapted for perform this filtering.

Once the final weight has been measured (user is quite enough and with reasonable angle form the horizontal), the CPU may compute the weight difference between after and before urination and store in non-volatile memory the weight with the time and date of the measurement. Optionally, the CPU may store the whole curve for further analysis by the person who will analyse the data.
- Once the weight difference between the after and before urination has been computed, it may be transformed by the CPU into a volume and the urinated volume (or weight) may be confirmed and displayed.
- The user can now shut the system down and release the disposable part.

It is to be understood that although preferred embodiments, specific constructions and configurations, as well as materials, have been discussed herein for devices according to the present invention, various changes or modifications in form and detail may be made without departing from the scope of this invention. Steps may be added or deleted to methods described within the scope of the present invention.

## Claims

1. A portable urination weight measurement device (1) comprising:
- a holding unit (11) for releasably holding a container (2), and
- a handle (12) comprising a weight measurement unit (121, 122) coupled to the holding unit (11) in such a way as to allow the measure of a weight exerted on the holding unit (11), the weight measurement unit comprising:
∘ a strain gauge (121), and
∘ an electronic circuit (122) suitable for converting a strain measurement into a weight signal and further adapted for determining a tilt angle of the portable urination weight measurement device (1) so as to compensate for said tilt during conversion of the strain measurement into the weight signal and/or so as to inform a user of the tilt or of an information derived therefrom,
wherein the strain gauge (121) is coupled to the holding unit (11) in such a way as to allow the measurement of a strain caused by a weight exerted on the holding unit (11) and is coupled to the electronic circuit (122) in such a way as to allow converting the strain measurement into a weight signal.

2. The portable urination weight measurement device (1) according to claim 1, wherein the handle (12) further comprises a release mechanism (126) coupled to the holding unit (11) in such a way as to allow the release of the container (2).

3. The portable urination weight measurement device (1) according to claim 1 or claim 2, wherein the handle (12) comprises a first surface (123) for resting on a toilet seat and wherein the holding unit (11) is suitable for extending downward when the surface (123) for resting on a toilet seat is resting on a toilet seat.

4. The portable urination weight measurement device (1) according to claim 3, wherein the surface (123) for resting on a toilet seat comprises means (1231) for hindering movements of the surface (123) when in contact with the toilet seat.

5. The portable urination weight measurement device (2) according to any of the preceding claims, wherein the holding unit (11) comprises a jaw (111) comprising two opposing parts (1111, 1112) that close for holding the container (2).

6. The portable urination weight measurement device (1) according to claim 5, wherein each opposing part (1111, 1112) is a flat plate.

7. The portable urination weight measurement device according to any one of the preceding claims, wherein the holding unit (11) is rotatably attached to the handle (12) in such a way that the portable device (1) can be folded by covering a second surface (124) of the handle (12) with a surface (112) of the holding unit (11).

8. The portable urination weight measurement device (1) according to any one of the preceding claims, wherein the electronic circuit (122) is further suitable for measuring the time at which the weight signal is measured.

9. The portable urination weight measurement device (1) according to any one of the preceding claims, wherein the electronic circuit (122) is suitable for sending:
a. the measured weight signal or data derived therefrom, and optionally
b. the time at which the weight signal is measured and the duration of the measurement,
to a remote receiver.

10. A toilet flushable container (2) having:
o a rim (21) delimiting its opening and having a fold (212) at a front side (f) of the opening and a fold (211) at a back side (b) of the opening, opposite to the fold (212) at the front side (f), each fold (211, 212) forming at rest an angle of less than 20°, and
o a collar (22) secured to the rim (21), the collar (22) comprising at least one fold (222) at the front side (f) of the opening forming at rest an angle of less than 20°, the collar (22) being of a rigidity such that increasing the angle of the at least one fold (222) to 180° increases the angle of the rim fold (211) at the back (b) of the opening to from 50 to 90°.

11. The toilet flushable container (2) according to claim 10, being water-soluble and suitable for containing fresh human urine for 40 seconds without leaking.

12. The toilet flushable container (2) according to claim 10 or claim 11, wherein the container (1) has a capacity of from 600 ml to 1000 ml, preferably from 600 ml to 700 ml.

13. The toilet flushable container (2) according to any one of claims 10 to 12, comprising means (23) for attaching to a holding unit (11) without penetration of the holding unit (11) in the container (2).

14. The toilet flushable container (2) according to any one of claims 10 to 13 comprising a guard (24) for protecting a holding unit (11) from urine projections.

15. A kit of parts comprising:
a. A portable urination weight measurement device (1) according to any one of claims 1 to 9, and
b. A toilet flushable container (2) according to any one of claims 10 to 14.
